# EUROPEAN PATENT SPECIFICATION

(11) **EP 2 948 771 B1**
(45) Date of publication and mention of the grant of the patent: **10.04.2019**
(21) Application number: 13872925.6
(22) Date of filing: 25.01.2013
(51) Int. Cl.: G01N 21/65, G01N 33/52, G01N 33/543, B82Y 15/00

(54) **CHEMICAL SENSING DEVICE**
CHEMISCHE ABTASTVORRICHTUNG
DISPOSITIF DE DÉTECTION CHIMIQUE

(43) Date of publication of application: 02.12.2015
(73) Proprietor: Hewlett-Packard Development Company L.P., Houston, TX 77070 (US)
(72) Inventor: ZHOU, Zhang-Lin, Palo Alto California 94304-1100 (US); LI, Zhiyong, Palo Alto California 94304-1100 (US); KIM, Ansoon, Palo Alto California 94304-1100 (US)
(74) Representative: HGF Limited
(86) International application number: PCT/US2013/023218
(87) International publication number: WO 2014/116234

(56) References cited:
- WO-A1-2012/054027
- WO-A1-2012/161683
- WO-A2-2012/079018
- US-A1- 2005 040 417
- MIN HU ET AL: "Gold Nanofingers for Molecule Trapping and Detection", JOURNAL OF THE AMERICAN CHEMICAL SOCIETY, vol. 132, no. 37, 22 September 2010 (2010-09-22), pages 12820-12822, XP055066395, ISSN: 0002-7863, DOI: 10.1021/ja105248h
- KLUTSE CHARLES K ET AL: "Optimization of SAM-based multilayer SERS substrates for intracellular analyses: the effect of terminating functional groups", SMART BIOMEDICAL AND PHYSIOLOGICAL SENSOR TECHNOLOGY VIII, SPIE, 1000 20TH ST. BELLINGHAM WA 98225-6705 USA, vol. 8025, no. 1, 13 May 2011 (2011-05-13) , pages 1-11, XP060016783, DOI: 10.1117/12.882309
- MEGAN E PEARCE ET AL: "Multifunctional Nanorods for Biomedical Applications", PHARMACEUTICAL RESEARCH, KLUWER ACADEMIC PUBLISHERS-PLENUM PUBLISHERS, NL, vol. 24, no. 12, 8 August 2007 (2007-08-08), pages 2335-2352, XP019555914, ISSN: 1573-904X, DOI: 10.1007/S11095-007-9380-7
- CHAPMAN ET AL.: 'Differentially ligand-functionalized microcantilever arrays for metal ion identification and sensing' ANALYTICAL CHEMISTRY vol. 79, no. 18, 2007, pages 7062 - 7068, XP055211950
- PATTON ET AL.: 'Rapid response microsensor for hydrogen detection using nanos tructured palladium films' SENSORS AND ACTUATORS A: PHYSICAL vol. 163, no. 2, 2010, pages 464 - 470, XP027452616
- KO ET AL.: 'Nanostructured surfaces and assemblies as SERS media' SMALL vol. 4, no. 10, 2008, pages 1576 - 1599, XP055193001
- "Nanostructure" In: "Nanostructure", 19 May 2016 (2016-05-19), XP055273743,
- KHLEBTSOV N G ET AL: "Optical properties and biomedical applications of plasmonic nanoparticles", JOURNAL OF QUANTITATIVE SPECTROSCOPY AND RADIATIVE TRANSFER, ELSEVIER SCIENCE, OXFORD, GB, vol. 111, no. 1, 1 January 2010 (2010-01-01), pages 1-35, XP026709764, ISSN: 0022-4073, DOI: 10.1016/J.JQSRT.2009.07.012 [retrieved on 2009-08-04]
- MALINSKY M D ET AL: "CHAIN LENGTH DEPENDENCE AND SENSING CAPABILITIES OF THE LOCALIZED SURFACE PLASMON RESONANCE OF SILVER NANOPARTICLES CHEMICALLY MODIFIED WITH ALKANETHIOL SELF-ASSEMBLED MONOLAYERS", JOURNAL OF THE AMERICAN CHEMICAL SOCIETY, AMERICAN CHEMICAL SOCIETY, US, vol. 123, 30 January 2001 (2001-01-30), pages 1471-1482, XP008067557, ISSN: 0002-7863, DOI: 10.1021/JA003312A
- Carly S. Levin ET AL: "Chain-Length-Dependent Vibrational Resonances in Alkanethiol Self-Assembled Monolayers Observed on Plasmonic Nanoparticle Substrates", Nano Letters, vol. 6, no. 11, 1 November 2006 (2006-11-01), pages 2617-2621, XP055325476, US ISSN: 1530-6984, DOI: 10.1021/nl062283k

## Description

### BACKGROUND

Systems for performing molecular analysis can include the use of surface-enhanced Raman spectroscopy (SERS), enhanced fluorescence, enhanced luminescence, and plasmonic sensing, among others. With specific regard to SERS, Raman spectroscopy is a spectroscopic technique used in condensed matter physics and chemistry to study various low-frequency modes in molecular systems. In further detail, in a Raman spectroscopic, an approximately monochromatic beam of light of a particular wavelength range passes through a sample of molecules and a spectrum of scattered light is emitted. The spectrum of wavelengths emitted from the molecule is called a "Raman spectrum" and the emitted light is called "Raman scattered light." A Raman spectrum can reveal electronic, vibrational, and rotational energies levels of a molecule. Different molecules produce different Ra-man spectrums that can be used like a fingerprint to identify molecules and even determine the structure of molecules. With this and other sensing techniques, enhancing device sensitivity, simplifying sensors, providing additional flexibility, etc., in such devices would be desirable.
Min Hu et al, Journal of the American Chemical Society, vol 132, no. 37, pages 12820-12822 discloses a molecular trap structure that can be formed to capture analyte molecules in solution for detection and identification. The structure is based on gold-coated nanoscale polymer fingers made by nanoimprinting technique.
WO 2012/079018 A2 discloses a surface plasmon sensor having an array of shaped protuberances with a metallic surface.
Klutse et al, Smart Biomedical and Physiological Sensor Technology VIII, SPIE, vol 8025, no. 1, pages 1-11 discloses multilayer SERS substrates comprising alternating layers of metal film and dielectric spacer cast on a monolayer of nanostructures.
Khlebtsov, et al, "Optical properties and biomedical applications of plasmonic nanoparticles", Journal of quantitative spectroscopy and radiative transfer, vol. 111, no. 1, 1 January 2010, pages 1-35 discloses with the optical properties and biomedical applications of plasmonic nanoparticles.
Malinsky, et al: "Chain length dependence and sensing capabilities of localized surface plasmon resonance of silver nanoparticles chemically modified with alkanethiol self-assembled monolayers", journal of the American chemical society, vol. 123, 30 January 2000, pages 1471-1482 discusses the optical properties of Ag nanoparticles chemically modified with alkanethiol.
Carly S. Levin, et al: "Chain-length-dependent vibrational resonances in Alkanethiol self-assembled monolayers observed on plasmonic nanoparticle substrates", Nano letters, vol. 6 no. 11, 1 November 2006, pages 2671-2621, relates to an alkanethiol self-assembled monolayer (SAM) on a gold surface.

### BRIEF DESCRIPTION OF THE DRAWINGS

Additional features and advantages of the invention will be apparent from the detailed description which follows, taken in conjunction with the accompanying drawings, which together illustrate, by way of example, features of the disclosure.
FIG. 1 is a cross-sectional view of a chemical sensing device in accordance with an example of the present disclosure;
FIG. 2 is a cross-sectional view of a chemical sensing device in accordance with an example of the present disclosure;
FIG. 3 is a perspective view of a chemical sensing device in accordance with an example of the present disclosure; and
FIG. 4 is a flow chart of a method in accordance with an example of the present disclosure; and
FIG. 5 is a flow chart of a method in accordance with an example of the present disclosure; and
FIG. 6 is a SERS spectra of a chemical sensing device exposed to Cr(VI) in accordance with an example of the present disclosure.

Reference will now be made to the exemplary embodiments illustrated, and specific language will be used herein to describe the same. It will nevertheless be understood that no limitation of the scope of the disclosure is thereby intended. The invention is defined in the appended claims. Only those passages for the following text which are in full agreement with the definitions of the independent claims relate to embodiments of the invention. All other statements merely relate to comparative examples.

### DETAILED DESCRIPTION

Raman spectroscopy can be used to study the transitions between molecular energy states when photons interact with molecules, which results in the energy of the scattered photons being shifted. The Raman scattering of a molecule can be seen as two processes. The molecule, which is at a certain energy state, is first excited into another (either virtual or real) energy state by the incident photons, which is ordinarily in the optical frequency domain. The excited molecule then radiates as a dipole source under the influence of the environment in which it sits at a frequency that may be relatively low (i.e., Stokes scattering), or that may be relatively high (i.e., anti-Stokes scattering) compared to the excitation photons. The Raman spectrum of different molecules or matters has characteristic peaks that can be used to identify the species. As such, Raman spectroscopy is a useful technique for a variety of chemical or biological sensing applications. However, the intrinsic Raman scattering process is very inefficient, and rough metal surfaces, various types of nano-antennas, as well as waveguiding structures have been used to enhance the Raman scattering processes (i.e., the excitation and/or radiation process described above).

The Raman scattered light generated by a compound (or ion) adsorbed on or within a few nanometers of a structured metal surface can be over 100 times greater than the Raman scattered light generated by the same compound in solution or in the gas phase. This process of analyzing a compound is called surface-enhanced Raman spectroscopy ("SERS"). In recent years, SERS has emerged as a routine and powerful tool for investigating molecular structures and characterizing interfacial and thin-film systems, and even enables single-molecule detection. Engineers, physicists, and chemists continue to seek improvements in systems and methods for performing SERS.

Most SERS systems only enhance the electro-magnetic field at certain hot spots. While this can be desirable, in many cases, the analytes are spread evenly on the SERS substrate, such as by simple adsorption. However, only a small fraction of the analytes actually populates the hot spots.

It has been recognized that it would be advantageous to develop a chemical sensing device based on a new class of surface-enhanced Raman spectroscopy (SERS) structures. Specifically, the present devices can contain a plurality of elongated nanostructures affixed to a substrate with a free end having a metallic coating or cap. The present nanostructures can flex and trap molecules which can then be sensed using SERS techniques. Further, the present nanostructures generally include ligands attached to the metallic coating or cap that can provide selectivity and sensitivity previously unachieved.

It is noted that when discussing a chemical sensing device, a method detecting a target molecule, or a method of making a chemical sensing device, each of these discussions can be considered applicable to the other embodiment, whether or not they are explicitly discussed in the context of that embodiment. Thus, for example, in discussing a ligand for a chemical sensing device, such a ligand can also be used in a method of detecting a target molecule, and *vice versa.*

Thus, a chemical sensing device can include a substrate and an elongated nanostructure having an attachment end and a free end opposite the attachment end, the attachment end affixed to the substrate and the free end including a metal having a potential sensing ligand attached thereto via a covalent bond.

As used herein, the term "nanostructure" refers to any structure having dimensions of width or diameter less than 1 micron. As such, an elongated nanostructure can include structures that have an aspect ratio with a length at least two times longer than the shortest width. Examples can include nanocones, nanopyramids, nanorods, nanobars, nanofingers, nanopoles and nanograss, without limitation thereto. As used herein, the terms "nanocones," "nanopyramids," "nanorods," "nanobars," "nanopoles" and "nanograss," refer to structures that are substantially: conical, pyramidal, rod-like, bar-like, pole-like and grass-like, respectively, which have nano-dimensions as small as a few tens of nanometers (nm) in height and a few nanometers in diameter, or width. For example, flexible columns may include nano-columns having the following dimensions: a diameter of 10 nm to 500 nm, a height of 20 nm to 2 micrometers (µm), and a gap between flexible columns of 20 nm to 500 nm. The terms of art, "substantially conical," "substantially pyramidal," "substantially rod-like," "substantially bar-like," "substantially pole-like" and "substantially grass-like," refers to structures that have nearly the respective shapes of cones, pyramids, rods, bars, poles and grass-like asperities within the limits of fabrication with nanotechnology.

As used herein, the term "metallic cap" refers to nanostructures, including nanospheres, prolate nanoellipsoids, oblate nanoellipsoids, nanodisks, and nanoplates, having a width or diameter of 500 nm or less. In one example, the metallic cap may possess shape-induced magnetic anisotropy. As used herein, the terms "nanospheres," "prolate nanoellipsoids," "oblate nanoellipsoids," "nanodisks," and "nanoplates," refer to structures that are substantially: spherical, prolate ellipsoidal, oblate ellipsoidal, disk-like, and plate-like, respectively, which have nano-dimensions as small as a few nanometers in size: height, diameter, or width. In addition, the terms "substantially spherical," "substantially prolate ellipsoidal," "substantially oblate ellipsoidal," "substantially disk-like," and "substantially and plate-like," refers to structures that have nearly the respective shapes of spheres, prolate ellipsoids, oblate ellipsoids, disks, and plates within the limits of fabrication with nanotechnology.

Generally, the elongated nanostructure can include a non-metallic column with a metallic coating or metallic cap. In one example, the nanostructure can include a polymer, such as a resist, coated with a SERS-active metal, such as gold, silver, copper, platinum, aluminum, etc. or the combination of those metals in the form of alloys. Generally, the SERS active metal can be selectively coated on the tips of the non-metallic column or deposited thereon. In addition, the SERS active metal can be a multilayer structure, for example, 10 to 100 nm silver layer with 1 to 50 nm gold over-coating, or vice versa. Additionally, the SERS active metal can be further coated with a thin dielectric layer.

Generally, the use of a polymer can render the nanostructures sufficiently flexible to permit the bending so that the tips meet at the top of the structure. Examples of suitable polymers include, but are not limited to, polymethyl methacrylate (PMMA), polycarbonate, siloxane, polydimethylsiloxane (PDMS), photoresist, nanoimprint resist, and other thermoplastic polymers and UV curable materials including one or more monomers/oligomers/polymers. In another example, the nanostructures can include an inorganic material having sufficient flexibility to bend. Examples of such inorganic materials include silicon oxide, silicon, silicon nitride, alumina, diamond, diamond-like carbon, aluminum, copper, and the like.

The chemical sensing device generally includes potential sensing ligands attached to the metallic coating or cap. In one example, the potential sensing ligand can include an attachment functional group(A), a spacer group (B), and a potential sensing moiety (PS) according to Formula I:

A-B-PS (I) Formula I

where A is an organic functional group attached to the nanostructure, B is substituted or unsubstituted, linear or branched alkyl or aryl, and PS is an organic functional group capable of binding to a target molecule. The functional group can include any group that is capable of covalent bonding to the metal coating or cap. The potential sensing moiety can include any moiety that is capable of interacting (including ionic, coordinate, covalent bonding) with a target molecule. The spacer group generally includes any group of atoms that covalently bond the functional group to the potential sensing moiety. For example, in one aspect, the metal can include gold and the functional group can include a thiol that covalently bonds to the gold. The ligand can further include an alkyl spacer group with an amine potential sensing moiety. For example, without be limiting, 3-aminopropylthiol would qualify as such a ligand, although other aminoalkylthiols would also qualify. Various functional groups can include primary amines, secondary amines, tertiary amines, amides, nitriles, isonitriles, cyanates, isocyanates, thiocyanates, isothiocyanates, azides, thiols, thiolates, sulfides, sulfinates, sulfonates, phosphates, hydroxyls, alcoholates, phenolates, carbonyls, carboxylates, phosphines, phosphine oxides, phosphonic acids, phosphoramides and phosphates. Additionally, various potential sensing groups can include functional groups as discussed herein, alone, or in combination with more complex structures, e.g., methyl red dyes, carboxyfluorescein dyes, carboxyrhodamine dyes, cyanine dyes, crown-ethers, polyamines etc. Particularly, a list of examples of potential sensing groups includes 4-pyridinethiol, 3-pyridinethiol, 2-pyridinethiol, 4-pyridinemethanethiol, 3-pyridinemethanethiol, 2-pyridinemethanethiol, 4-pyridineethanethiol, 3-pyridineethanethiol, 2-pyridineethanethiol, 4-pyridinepropanethiol, 3-pyridinepropanethiol, 2-pyridinepropanethiol, 4-pyridinebutanethiol, 3-pyridinebutanethiol, 2-pyridinebutanethiol, 4-pyridinepentanethiol, 3-pyridinepentanethiol, 2-pyridinepentanethiol, etc.

The chemical sensing device generally includes a potential sensing ligand formulated to selectively bind a target molecule. The target molecule can be a metal ion, an organic compound, or a hydrogen ion. In one example, the target molecule is a metal ion and the potential sensing ligand is formulated to selectively bind the metal ion. In one example, the metal ions can include chromium, lead, mercury, zinc, calcium, sodium, hydrogen, potassium, arsonium, and mixtures thereof.

By using the present potential sensing ligands, the chemical sensing device can be sensitive enough to detect a target molecule; including a metal ion, an organic compound, or a hydrogen ion, at a concentration as low as 1 part-per-million (ppm). In one aspect, the sensitivity can be as low as 1 part-per-billion (ppb), and in one specific aspect, as low as 1 part-per-trillion (ppt).

Regarding sensitivity, the present chemical device can include a plurality of elongated nanostructures attached to a substrate forming an array. In one example, the array can include sub-arrays. In one aspect, the sub-arrays can each have individual selectivity for a target molecule. As such, one array can have selectivity for a plurality of target molecules. The chemical sensing device can be further configured to detect the target molecule from a liquid or gas.

Additionally, the chemical sensing device can further include a detector operatively coupled to the nanostructure. In one example, the detector can be a colorimeter, a reflectometer, a spectrometer, a spectrophotometer, a Raman spectrometer, an optical microscope, and/or an instrument for measuring luminescence.

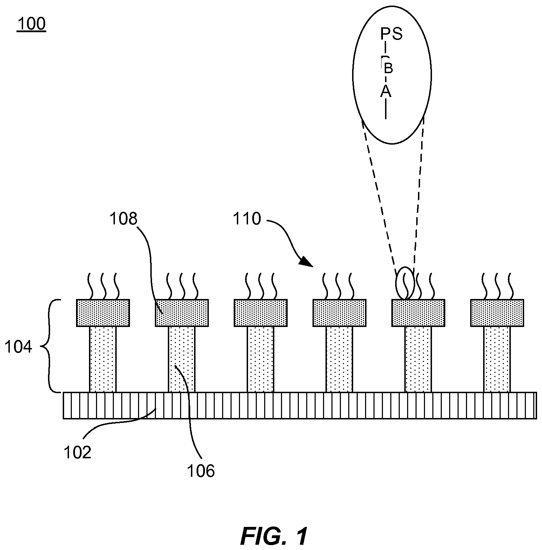

Referring to FIG. 1, a chemical sensing device 100 can include a substrate 102 having an elongated nanostructure 104 attached thereto. The elongated nanostructure has a columnar structure 106 with a metallic cap 108 deposited thereon. Further, potential sensing ligands 110 can be covalently bonded to the metallic cap. As shown in the insert, the potential sensing ligands can generally includes the structure A-B-PS, where A is a functional group that binds the ligand to the metallic cap, B is a spacer group, and PS is a potential sensing moiety coupled to the functional group through the spacer group. While the present figure provides a specific structure of the chemical sensing device, it is understood that the illustrated structure is not intended to be limiting and that the present disclosure contemplates the use of various elements as discussed herein. For example, the present metallic cap could also be a metallic coating.

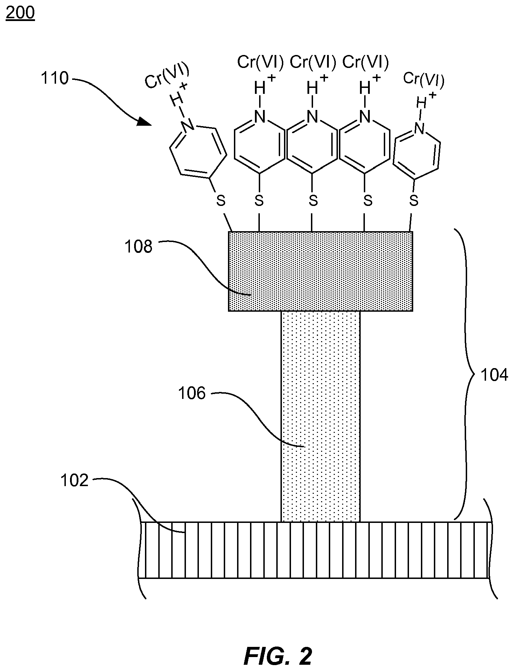

Referring to FIG. 2, an expanded view of a single elongated nanostructure having ligands bonded thereto is illustrated. It is noted that the elements of FIG. 2 are not necessarily drawn to scale, nor does it represent every chemical sensing device available for use herein, i.e. it provides merely an exemplary embodiment of one chemical sensing device having one specific set of ligands. In this example, the chemical sensing device 200 can include a substrate 102 having an elongated nanostructure 104 attached thereto. The elongated nanostructure can include a columnar structure 106 with a metallic cap 108 deposited thereon. A plurality of pyridinium potential sensing moieties 110 are attached to the metallic cap via a thiol linkage. The pyridinium potential sensing moieties are shown as binding to chromium (VI) in this specific example.

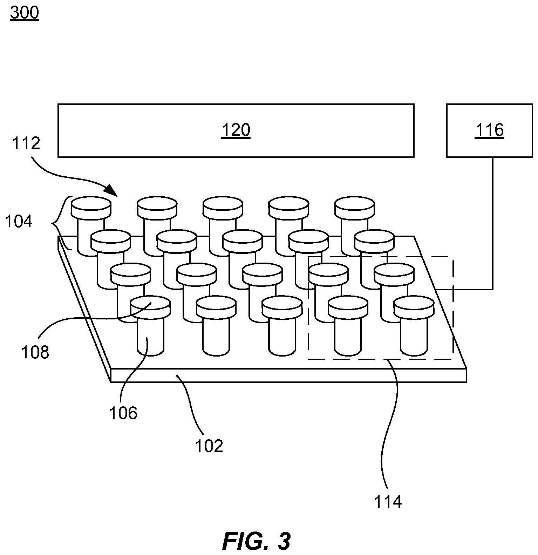

Referring to FIG. 3, a chemical sensing device 300 can include a substrate 102 having an elongated nanostructures 104 attached thereto. The elongated nanostructures can include columnar structures 106 with a metallic caps 108 deposited thereon. The metallic caps can have ligands (not shown) attached thereto as well. The plurality of nanostructures can form an array 112 with a sub-array 114. The chemical sensing device can further include a detector 116 operatively coupled to the nanostructures. Additionally, a source of excitation energy 120, such as a light source or a laser source, is also shown.

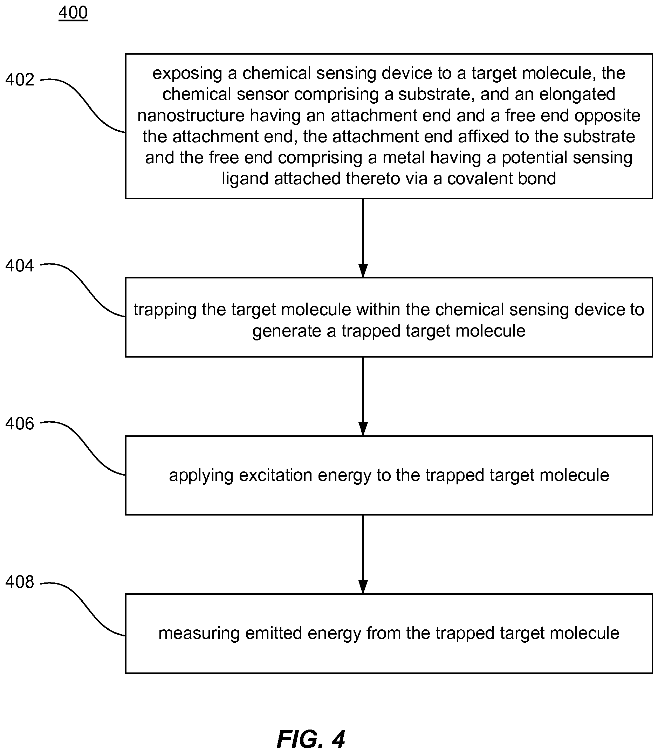

Referring to FIG. 4, a method for detecting a target molecule 400 can include exposing 402 a chemical sensing device to a target molecule, the chemical sensing device including any of those described herein, trapping 404 the target molecule within the chemical sensing device to generate a trapped target molecule; applying 406 excitation energy to the trapped target molecule; and measuring 408 emitted energy from the trapped target molecule. In one example, the excitation energy and the emitted energy can be electromagnetic energy. Additionally, the method can further include flushing the trapped metal target molecule from the chemical sensing device. As such, the present devices can be reusable and/or can be recyclable.

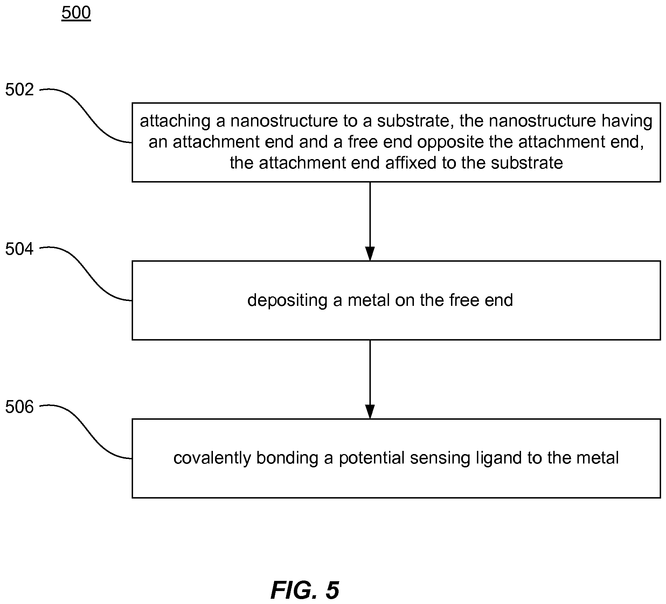

Referring to FIG. 5, a method of making a chemical sensing device 500 can include disposing 502 a nanostructure to a substrate, the nanostructure having an attachment end attached to the substrate and a free end opposite the attachment end, the attachment end affixed to the substrate; depositing 504 a metal on the free end; and covalently bonding 506 a potential sensing ligand to the metal.

It is noted that, as used in this specification and the appended claims, the singular forms "a," "an," and "the" include plural referents unless the context clearly dictates otherwise.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 wt% to about 5 wt%" should be interpreted to include not only the explicitly recited values of about 1 wt% to about 5 wt%, but also include individual values and sub-ranges within the indicated range. Thus, included in this numerical range are individual values such as 2, 3.5, and 4 and sub-ranges such as from 1-3, from 2-4, and from 3-5, etc. This same principle applies to ranges reciting only one numerical value. Furthermore, such an interpretation should apply regardless of the breadth of the range or the characteristics being described.

### EXAMPLES

The following examples illustrate embodiments of the disclosure that are presently known. Thus, these examples should not be considered as limitations of the invention, but are merely in place to teach how to make devices of the present disclosure. As such, a representative number of devices and their method of manufacture are disclosed herein.

### Example 1 - General Preparation Method for Modifying Gold Elongated Nanostructure with Potential Sensing Ligand

Immobilizing a potential sensing moiety to a gold surface on a nanostructure can be carried out with surface modification by treating the gold nanostructure with chemical reagents. A reactant moiety A is introduced on the surface. Additionally, a reactant moiety B having a potential sensing (PS) moiety bound thereto is reacted with the reactant moiety A. For example, the modified substrate can be dipped into the solutions of potential sensing moieties bound to the reactant moiety B. The reactant moieties A and B react heterogeneously but neither react homogeneously to form a covalent bond between the substrate and the potential sensor moiety, thus immobilizing the potential chemical sensing moiety onto the gold coated nanostructure surface.

### Example 2 - Preparation of Gold Elongated Nanostructure with Potential Sensing Ligand

Surface modification of a gold nanostructure is performed with 3-aminopropylthiol providing a modified surface with a reactive amino group, which is reactive towards an activated ester. The modified gold nanostructure is then treated with a solution of the activated ester, where the activated ester includes a potential sensing moiety. The reaction between the activated ester and the amino group takes place, forming a covalent bond between the potential sensing molecular moiety and the gold nanostructure surface via the propylthiol linkage.

### Example 3 - Preparation of Gold Elongated Nanostructure with Potential Sensing Ligand

**The** present example sets for the preparation of a nanostructure modified with a pH indicator to sense acidity. First, commercially available methyl red is modified with DSC (N,N'-disuccinimidyl carbonate) in the presence of catalytic amount of 3-dimethylaminopyridine to give modified methyl red with reactive succinimidyl group. Second, a gold nanostructure is modified with the solution of 3-aminopropylthiol, generating a modified gold nanostructure with a reactive amino group on the surface. When the modified nanofinger is treated with the modified methyl red, a reaction between the surface and the dye takes place, forming a gold nanofinger attached with methyl red dye, which can be used for detecting proton ion H⁺.

### Example 4 - Alternative General Preparation Method for Modifying Gold Elongated Nanostructure with Potential Sensing Ligand

Another approach is to first modify the potential sensing moiety with certain chemical reagents to form a modified potential sensing moiety with reactive groups, which are reactive towards gold nanostructure surfaces. For example, an activated ester having a potential sensing moiety can be treated with commercially available 3-aminopropylthiol, and the reaction between the amino group with the activated ester takes place, generating a modified potential sensing moiety coupled to a reactive thiol group, which is reactive towards gold nanostructure surface. When the untreated gold nanostructure is treated with the modified potential sensing moiety coupled to a reactive thiol group, a chemical reaction between the thiol group and gold surface takes place to form a chemical bond, thus immobilizing the potential sensing moiety on the gold nanostructure surface.

### Example 5 - Preparation of Gold Elongated Nanostructure with Potential Sensing Ligand

Reaction of methyl red dye with 3-aminopropylthiol in the presence of coupling reagent such as N.N'-dicyclohexylcarbodiimide (DCC) forms a modified methyl red with thiol group on the end, which is reactive towards a gold surface. When a gold nanostructure is treated with the modified methyl red, a covalent bond forms between the gold nanostructure and methyl red. Thus, the methyl dye is immobilized on the gold nanostructure surface, which can be used for detection of proton ions H⁺.

### Example 6 - Preparation of Gold Elongated Nanostructure with Potential Sensing Ligand

Nanostructures (referred to as "NS" in Scheme 1 below) can be used to detect metal ions, such as chromium(VI). As shown in Scheme 1: Reaction of 4-vinylpyridine with thiourea in the presence of p-toluenesulfonic acid gave intermediate 2. Treatment of intermediate 2 with ammonium hydroxide formed 4-pyridineethantiol 3, which can be converted into HCI salt 4. Upon careful neutralization of HCI salt 4 with a base, it can be self-assembled into a gold nanostructure (NS) surface. Since the pyridine can form a coordination bond with Cr (VI), it can be used for highly sensitive and selective detection of chromium (VI).

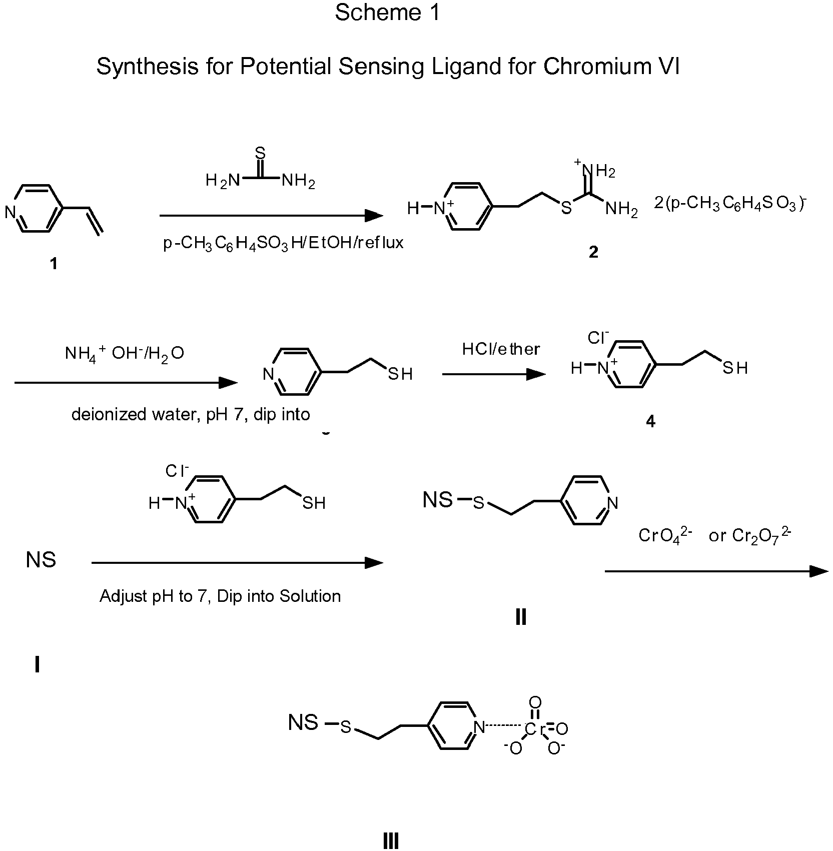

### Example 7 - Preparation of Gold Elongated Nanostructure with Potential Sensing Ligand

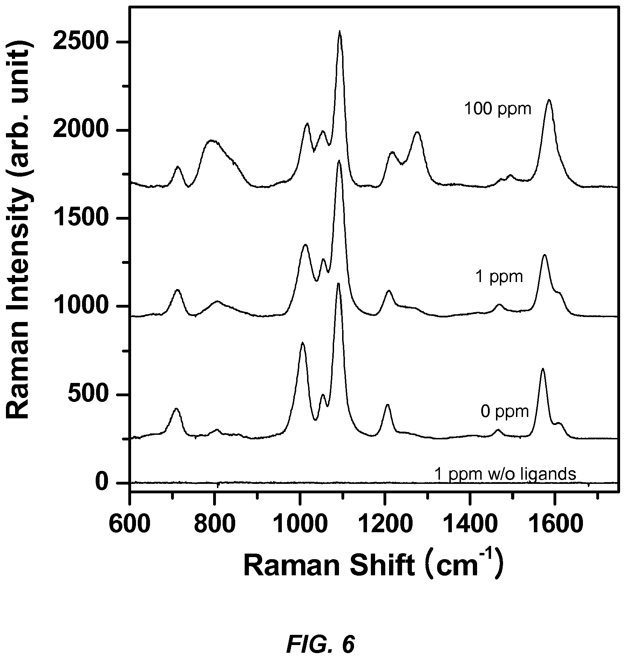

A gold nanostructure was prepared by treatment of the surface of the gold with 4-mercaptopyridine. A gold nanostructure was dipped without stirring into a solution of ethanol containing 4-mercaptopyridine for 24 hrs at room temperature, followed by being rinsed with pure ethanol solvent. In order to prepare a cationic pyridinium form of the functionalized 4-mercaptopyridine, the gold nanostructure was re-dipped in 0.1 M sulfuric acid for 30 min. The pyridinium-functionalized nanostructure was then exposed to varying amount of Cr(VI) as shown in FIG. 6. Notably, the SERS detected the presence of Cr(VI) at concentrations as low as 1 ppm. Further, based on the data obtained, lower thresholds appear possible as the spectra is clearly resolvable between 0 ppm and 1 ppm. As a comparison, the bottom flat line represents the same nanostructure exposed to Cr(VI), except no ligands were attached thereto. Notably, the spectra show no activity. The evidence provided in FIG. 6 shows that the present ligands provide detectability of a metal ion otherwise unachieved, with a sensitivity down to 1 ppm.

While the invention has been described with reference to certain examples, those skilled in the art will appreciate that various modifications, changes, omissions, and substitutions can be made without departing from the scope of the following claims.

## Claims

1. A chemical sensing device (100), comprising:
a substrate (102); and
a plurality of elongated nanostructures (104), said elongated nanostructures (104) having ar attachment end and a free end opposite the attachment end, the attachment end affixed to the substrate;
wherein each of the elongated nanostructures (104) is a structure that has an aspect ratio with a length of at least two times longer than the shortest width: wherein said elongated nanostructures (104) comprise nanocones, nanopyramids, nanorods, nanobars, nanofingers, nanopoles or nanograss; and wherein the term nanostructure refers to any structure having dimensions of width or diameter less than 1 micron;
wherein the nanostructures (104) are flexible and wherein the flexible nanostructures (104) are arranged so as to be able to flex and trap molecules;
**characterized in that** the free end comprises a metallic cap (108) having a potential sensing ligand (110) attached thereto via a covalent bond; and
the potential sensing ligand (110) comprises an attachment functional group(A), a spacer group (B), and a potential sensing moiety (PS) according to formula I:
A-B-PS (I)
wherein A is an organic functional group attached to the nanostructure, B is substituted or unsubstituted, linear or branched, alkyl or aryl, and PS is an organic functional group capable of binding to a target molecule.

2. The chemical sensing device (100) of claim 1, wherein the nanostructure (104) comprises a non-metallic column (106).

3. The chemical sensing device (100) of claim 1 , wherein the metal is selected from the group of: gold, silver, copper, aluminum, platinum, and mixtures thereof.

4. The chemical sensing device (100) of claim 1 , wherein the potential sensing ligand (110) is formulated to selectively bind a metal ion, an organic compound, or a hydrogen ion.

5. The chemical sensing device (100) of claim 4, wherein the potential sensing ligand is formulated to selectively bind the metal ion, and wherein the metal ion is selected from the group of: chromium, lead, mercury, zinc, calcium, sodium, hydrogen, potassium, arsonium, and mixtures thereof.

6. The chemical sensing device (100) of claim 4, wherein the chemical sensing device is sensitive enough to detect the metal ion, the organic compound, or the hydrogen ion at a concentration as low as 1 ppt.

7. The chemical sensing device (100) of claim 1 , further comprising a detector (116) operatively coupled to the nanostructure, the detector selected from the group of a colorimeter, a reflectometer, a spectrometer, a spectrophotometer, a Raman spectrometer, an optical microscope, and an instrument for measuring luminescence.

8. The chemical sensing device (100) of claim 1, further comprising a plurality of the elongated nanostructures attached to the substrate forming an array (112).

9. The chemical sensing device (100) of claim 8, wherein the array includes sub-arrays, the sub-arrays (114) having individual selectivity for a target molecule, the target molecule selected from the group of a metal ion, an organic compound, and a hydrogen ion.

10. The chemical sensing device (100) of claim 8, wherein the chemical sensing device (110) is adapted to detect the target molecule from a liquid or gas.

11. A method for detecting a target molecule, comprising exposing a chemical sensing device (100) to a target molecule, the chemical sensing device (100) comprising:
a substrate (102), and
a plurality of elongated nanostructures (104), said nanostructures (104) having an attachment end and a free end opposite the attachment end, the attachment end affixed to the substrate (102) and the free end comprising a metallic cap (108) having a potential sensing ligand (110) attached thereto via a covalent bond;
trapping the target molecule within the chemical sensing device (100) to generate a trapped target molecule;
applying excitation energy to the trapped target molecule; and
measuring emitted energy from the trapped target molecule;
wherein each of the elongated nanostructures (104) is a structure that has an aspect ratio with a length of at least two times longer than the shortest width: wherein said elongated nanostructures (104) comprise nanocones, nanopyramids, nanorods, nanobars, nanofingers, nanopoles or nanograss; and wherein the term nanostructure refers to any structure having dimensions of width or diameter less than 1 micron;
wherein the nanostructures (104) are flexible and wherein the flexible nanostructures (104) are arranged so as to be able to flex and trap molecules; and
wherein the potential sensing ligand (110) comprises an attachment functional group(A), a spacer group (B), and a potential sensing moiety (PS) according to formula I:
A-B-PS (I)
wherein A is an organic functional group attached to the nanostructure, B is substituted or unsubstituted, linear or branched, alkyl or aryl, and PS is an organic functional group capable of binding to a target molecule.

12. The method of claim 11, wherein the excitation energy and the emitted energy is electromagnetic energy and the target molecule is selected from the group of a metal ion, an organic compound, and a hydrogen ion.

13. The method of claim 11, further comprising flushing the trapped metal target molecule from the chemical sensing device (100).

14. A method of making a chemical sensing device (100) according to claim 1, comprising:
disposing a plurality of elongated nanostructures (104) on a substrate (102), the nanostructures having an attachment end attached to the substrate and a free end opposite the attachment end;
wherein each of the elongated nanostructures (104) is a structure that has an aspect ratio with a length of at least two times longer than the shortest width; wherein said elongated nanostructures (104) comprise nanocones, nanopyramids, nanorods, nanobars, nanofingers, nanopoles or nanograss; wherein the term nanostructure refers to any structure having dimensions of width or diameter less than 1 micron; and wherein the nanostructures (104) are flexible and wherein the flexible nanostructures (104) are arranged so as to be able to flex and trap molecules;
**characterized by** depositing a metal on the free end thereby forming a metallic cap (108); and
covalently bonding a potential sensing ligand (110) to the metal; wherein the potential sensing ligand (110) comprises an attachment functional group(A), a spacer group (B), and a potential sensing moiety (PS) according to formula I:
A-B-PS (I)
wherein A is an organic functional group attached to the nanostructure (104), B is substituted or unsubstituted, linear or branched, alkyl or aryl, and PS is an organic functional group capable of binding to a target molecule.

## Patentansprüche

1. Chemische Messvorrichtung (100), umfassend:
ein Substrat (102); und
mehrere längliche Nanostrukturen (104), wobei die länglichen Nanostrukturen (104) ein Befestigungsende und ein freies Ende gegenüber dem Befestigungsende aufweisen, wobei das Befestigungsende an dem Substrat befestigt ist;
wobei jede der länglichen Nanostrukturen (104) eine Struktur ist, die ein Aspektverhältnis aufweist, bei dem die Länge mindestens zweimal so lang wie die kürzeste Breite ist; wobei die länglichen Nanostrukturen (104) Nanokegel, Nanopyramiden, Nanostäbchen, Nanobalken, Nanofinger, Nanopole oder Nanogras umfassen; und wobei sich der Begriff "Nanostruktur" auf eine beliebige Struktur mit Abmessungen der Breite oder des Durchmessers von weniger als 1 Mikrometer bezieht;
wobei die Nanostrukturen (104) flexibel sind und wobei die flexiblen Nanostrukturen (104) derart angeordnet sind, dass sie Moleküle biegen und einfangen können;
**dadurch gekennzeichnet, dass** das freie Ende eine Metallkappe (108) mit einem potentiellen Messliganden (110) umfasst, der über eine kovalente Bindung daran befestigt ist; und
der potentielle Messligand (110) eine funktionelle Anbindungsgruppe (A), eine Spacergruppe (B) und eine potentielle Messeinheit (potential sensing moiety, PS) gemäß Formel I umfasst:
A-B-PS (I)
wobei A eine an die Nanostruktur gebundene organische funktionelle Gruppe ist, B substituiert oder unsubstituiert, linear oder verzweigt, Alkyl oder Aryl ist, und PS eine organische funktionelle Gruppe ist, die an ein Zielmolekül binden kann.

2. Chemische Messvorrichtung (100) nach Anspruch 1, wobei die Nanostruktur (104) eine nichtmetallische Säule (106) umfasst.

3. Chemische Messvorrichtung (100) nach Anspruch 1, wobei das Metall ausgewählt ist aus der Gruppe, bestehend aus:
Gold, Silber, Kupfer, Aluminium, Platin und Mischungen davon.

4. Chemische Messvorrichtung (100) nach Anspruch 1, wobei der potentielle Messligand (110) derart formuliert ist, dass er selektiv ein Metallion, eine organische Verbindung oder ein Wasserstoffion binden kann.

5. Chemische Messvorrichtung (100) nach Anspruch 4, wobei der potentielle Messligand derart formuliert ist, dass er selektiv das Metallion binden kann, und wobei das Metallion ausgewählt ist aus der Gruppe, bestehend aus: Chrom, Blei, Quecksilber, Zink, Calcium, Natrium, Wasserstoff, Kalium, Arsonium, und Mischungen davon.

6. Chemische Messvorrichtung (100) nach Anspruch 4, wobei die chemische Messvorrichtung sensibel genug ist, um das Metallion, die organische Verbindung, oder
das Wasserstoffion in einer Konzentration von nur 1 ppt zu erfassen.

7. Chemische Messvorrichtung (100) nach Anspruch 1, ferner umfassend einen Detektor (116), der operativ mit der Nanostruktur gekoppelt ist, wobei der Detektor aus der Gruppe eines Colorimeters, eines Reflektometers, eines Spektrometers, eines Spektralphotometers, eines Raman-Spektrometers, eines optischen Mikroskops und eines Instruments zur Messung der Lumineszenz ausgewählt ist.

8. Chemische Messvorrichtung (100) nach Anspruch 1, die ferner mehrere längliche Nanostrukturen umfasst, die an dem Substrat angebracht sind, um eine Anordnung (112) zu bilden.

9. Chemische Messvorrichtung (100) nach Anspruch 8, wobei die Anordnung Unteranordnungen enthält, wobei die Unteranordnungen (114) eine individuelle Selektivität für ein Zielmolekül aufweisen, wobei das Zielmolekül aus einer Gruppe aus einem Metallion, einer organischen Verbindung, und einem Wasserstoffion ausgewählt ist.

10. Chemische Messvorrichtung (100) nach Anspruch 8, wobei die chemische Messvorrichtung (110) angepasst ist, um das Zielmolekül aus einer Flüssigkeit oder einem Gas zu erfassen.

11. Verfahren zum Detektieren eines Zielmoleküls, umfassend:
Aussetzen einer chemischen Messvorrichtung (100) gegenüber einem Zielmolekül, wobei die chemische Messvorrichtung (100) Folgendes umfasst:
ein Substrat (102); und
mehrere längliche Nanostrukturen (104), wobei die länglichen Nanostrukturen (104) ein Befestigungsende und ein freies Ende gegenüber dem Befestigungsende aufweisen, wobei das Befestigungsende an dem Substrat (102) befestigt ist und das freie Ende eine metallische Kappe (108) umfasst, die einen potentiellen Messliganden (110) aufweist, der daran über eine kovalente Bindung befestigt ist;
Einschließen des Zielmoleküls mit der chemischen Messvorrichtung (100) um ein eingeschlossenes Zielmolekül zu erzeugen;
Anwenden von Erregungsenergie an das eingeschlossene Zielmolekül; und Messen von emittierter Energie aus dem eingeschlossenen Zielmolekül;
wobei jede der länglichen Nanostrukturen (104) eine Struktur ist, die ein Aspektverhältnis aufweist, bei dem die Länge mindestens zweimal so lang wie die kürzeste Breite ist; wobei die länglichen Nanostrukturen (104) Nanokegel, Nanopyramiden, Nanostäbchen, Nanobalken, Nanofinger, Nanopole oder Nanogras umfassen; und wobei sich der Begriff "Nanostruktur" auf eine beliebige Struktur mit Abmessungen der Breite oder des Durchmessers von weniger als 1 Mikrometer bezieht;
wobei die Nanostrukturen (104) flexibel sind und wobei die flexiblen Nanostrukturen (104) derart angeordnet sind, dass sie Moleküle biegen und einfangen können; und
wobei der potentielle Messligand (110) eine funktionelle Anbindungsgruppe (A),
eine Spacergruppe (B) und eine potentielle Messeinheit (PS) gemäß Formel I umfasst:
A-B-PS (I)
wobei A eine an die Nanostruktur gebundene organische funktionelle Gruppe ist, B substituiert oder unsubstituiert, linear oder verzweigt, Alkyl oder Aryl ist, und PS eine organische funktionelle Gruppe ist, die an ein Zielmolekül binden kann.

12. Verfahren nach Anspruch 11, wobei die Anregungsenergie und die emittierte Energie elektromagnetische Energie sind und das Zielmolekül aus einer Gruppe aus einem Metallion, einer organischen Verbindung, und einem Wasserstoffion ausgewählt ist.

13. Verfahren nach Anspruch 11, das ferner das Spülen des eingeschlossenen Metallzielmoleküls aus der chemischen Messvorrichtung (100) umfasst.

14. Verfahren zur Herstellung einer chemischen Messvorrichtung (100) nach Anspruch 1, umfassend:
Anordnen von mehreren länglichen Nanostrukturen (104) auf einem Substrat (102), wobei die Nanostrukturen ein Befestigungsende, das an dem Substrat befestigt ist, und ein freies Ende gegenüber dem Befestigungsende aufweisen, wobei jede der länglichen Nanostrukturen (104) eine Struktur ist, die ein Aspektverhältnis mit einer Länge aufweist, die mindestens zwei Mal länger als die kürzeste Breite ist; wobei die länglichen Nanostrukturen (104) Nanokegel, Nanopyramiden, Nanostäbchen, Nanobalken, Nanofinger, Nanopole oder Nanogras umfassen; wobei sich der Begriff Nanostruktur auf jede Struktur bezieht, deren Abmessungen in der Breite oder im Durchschnitt geringer als 1 Mikrometer sind; und wobei die Nanostrukturen (104) flexibel sind und wobei die flexiblen Nanostrukturen (104) derart angeordnet sind, dass sie Moleküle biegen und einfangen können;
**gekennzeichnet durch**
Abscheiden eines Metalls auf dem freien Ende, wodurch eine metallische Kappe (108) gebildet wird: und
kovalentes Binden eines potentiellen Messliganden (110) an das Metall; wobei der potentielle Messligand (110) eine funktionelle Anbindungsgruppe (A), eine Spacergruppe (B) und eine potentielle Messeinheit (PS) gemäß Formel I umfasst:
A-B-PS (I)
wobei A eine an die Nanostruktur (104) gebundene organische funktionelle Gruppe ist, B substituiert oder unsubstituiert, linear oder verzweigt, Alkyl oder Aryl ist, und PS eine organische funktionelle Gruppe ist, die an ein Zielmolekül binden kann.

## Revendications

1. Dispositif de détection chimique (100), comprenant :
un substrat (102) ; et
une pluralité de nanostructures allongées (104), lesdites nanostructures allongées (104) ayant une extrémité de fixation et une extrémité libre opposée à l'extrémité de fixation, l'extrémité de fixation étant fixée au substrat :
dans lequel chacune des nanostructures allongées (104) est une structure qui présente un rapport géométrique d'une longueur au moins deux fois plus longue que la largeur la plus courte ; dans lequel lesdites nanostructures allongées (104) comprennent des nanocônes, des nanopyramides, des nanotiges, des nanobarres, des nanodoigts, des nanopôles ou de la nanoherbe ; et dans lequel le terme nanostructure désigne n'importe quelle structure présentant des dimensions de largeur ou de diamètre inférieures à 1 micron ;
dans lequel les nanostructures (104) sont flexibles et dans lequel les nanostructures flexibles (104) sont agencées de manière à pouvoir plier et piéger des molécules ;
**caractérisé en ce que** l'extrémité libre comprend un capuchon métallique (108) comportant un ligand de détection de potentiel (110) fixé à celui-ci via une liaison covalente ; et
le ligand de détection de potentiel (110) comprend un groupe fonctionnel de fixation (A), un groupe d'espacement (B) et une fraction de détection de potentiel (PS) selon la formule I :
A-B-PS (I)
dans laquelle A est un groupe fonctionnel organique fixé à la nanostructure, B est un alkyle ou un aryle linéaire ou ramifié, substitué ou non substitué, et PS est un groupe fonctionnel organique capable de se lier à une molécule cible.

2. Dispositif de détection chimique (100) selon la revendication 1, dans lequel la nanostructure (104) comprend une colonne non métallique (106).

3. Dispositif de détection chimique (100) selon la revendication 1, dans lequel le métal est choisi dans le groupe composé de :
or, argent, cuivre, aluminium, platine et leurs mélanges.

4. Dispositif de détection chimique (100) selon la revendication 1, dans lequel le ligand de détection de potentiel (110) est formulé pour se lier de manière sélective à l'ion métallique, à un composé organique ou à un ion hydrogène.

5. Dispositif de détection chimique (100) selon la revendication 4, dans lequel le ligand de détection de potentiel est formulé pour se lier de manière sélective à l'ion métallique, et dans lequel l'ion métallique est choisi dans le groupe composé de : chrome, plomb, mercure, zinc, calcium, sodium, hydrogène, potassium, arsonium et leurs mélanges.

6. Dispositif de détection chimique (100) selon la revendication 4, dans lequel le dispositif de détection chimique est suffisamment sensible pour détecter l'ion métallique, le composé organique ou l'ion hydrogène à une concentration aussi basse que 1 ppt.

7. Dispositif de détection chimique (100) selon la revendication 1, comprenant en outre un détecteur (116) couplé de manière opérationnelle à la nanostructure, le détecteur étant choisi dans le groupe composé d'un colorimètre, d'un réflectomètre, d'un spectromètre, d'un spectrophotomètre, d'un spectromètre Raman, d'un microscope optique et d'un instrument de mesure de la luminescence.

8. Dispositif de détection chimique (100) selon la revendication 1, comprenant en outre une pluralité des nanostructures allongées fixées au substrat formant un réseau (112).

9. Dispositif de détection chimique (100) selon la revendication 8, dans lequel le réseau comprend des sous-réseaux, les sous-réseaux (114) ayant une sélectivité individuelle pour une molécule cible, la molécule cible étant choisie dans le groupe composé d'un ion métallique, d'un composé organique et d'un ion hydrogène.

10. Dispositif de détection chimique (100) selon la revendication 8, dans lequel le dispositif de détection chimique (110) est conçu pour détecter la molécule cible parmi un liquide ou un gaz.

11. Procédé de détection d'une molécule cible, comprenant l'exposition d'un dispositif de détection chimique (100) à une molécule cible, le dispositif de détection chimique (100) comprenant :
un substrat (102), et
une pluralité de nanostructures allongées (104), lesdites nanostructures (104) ayant une extrémité de fixation et une extrémité libre opposée à l'extrémité de fixation, l'extrémité de fixation étant fixée au substrat (102) et l'extrémité libre comprenant un capuchon métallique (108) comportant un ligand de détection de potentiel (110) fixé à celui-ci via une liaison covalente ;
le piégeage de la molécule cible à l'intérieur du dispositif de détection chimique (100) pour générer une molécule cible piégée ;
l'application d'une énergie d'excitation à la molécule cible piégée ; et
la mesure de l'énergie émise à partir de la molécule cible piégée ;
dans lequel chacune des nanostructures allongées (104) est une structure qui présente un rapport géométrique d'une longueur au moins deux fois plus longue que la largeur la plus courte ; dans lequel lesdites nanostructures allongées (104) comprennent des nanocônes, des nanopyramides, des nanotiges, des nanobarres, des nanodoigts, des nanopôles ou de la nanoherbe ; et dans lequel le terme nanostructure désigne n'importe quelle structure présentant des dimensions de largeur ou de diamètre inférieures à 1 micron ;
dans lequel les nanostructures (104) sont flexibles et dans lequel les nanostructures flexibles (104) sont agencées de manière à pouvoir plier et piéger des molécules ; et
dans lequel le ligand de détection de potentiel (110) comprend un groupe fonctionnel de fixation (A), un groupe d'espacement (B) et une fraction de détection de potentiel (PS) selon la formule I :
A-B-PS (I)
dans laquelle A est un groupe fonctionnel organique fixé à la nanostructure, B est un alkyle ou un aryle linéaire ou ramifié, substitué ou non substitué, et PS est un groupe fonctionnel organique capable de se lier à une molécule cible.

12. Procédé selon la revendication 11, dans lequel l'énergie d'excitation et l'énergie émise est de l'énergie électromagnétique et la molécule cible est choisie dans le groupe composé d'un ion métallique, d'un composé organique, et d'un ion hydrogène.

13. Procédé selon la revendication 11, comprenant en outre l'évacuation de la molécule cible métallique piégée du dispositif de détection chimique (100).

14. Procédé de fabrication d'un dispositif de détection chimique (100) selon la revendication 1, comprenant :
la disposition d'une pluralité de nanostructures allongées (104) sur un substrat (102), les nanostructures ayant une extrémité de fixation fixée au substrat et une extrémité libre opposée à l'extrémité de fixation ; dans lequel chacune des nanostructures allongées (104) est une structure qui présente un rapport géométrique d'une longueur au moins deux fois plus longue que la largeur la plus courte ; dans lequel lesdites nanostructures allongées (104) comprennent des nanocônes, des nanopyramides, des nanotiges, des nanobarres, des nanodoigts, des nanopôles ou de la nanoherbe ; dans lequel le terme nanostructure désigne n'importe quelle structure présentant des dimensions de largeur ou de diamètre inférieures à 1 micron ; et dans lequel les nanostructures (104) sont flexibles et dans lequel les nanostructures flexibles (104) sont agencées de manière à pouvoir plier et piéger des molécules ;
**caractérisé par**
le dépôt d'un métal sur l'extrémité libre formant ainsi un capuchon métallique (108) ; et
la liaison covalente d'un ligand de détection de potentiel (110) avec le métal ; dans lequel le ligand de détection de potentiel (110) comprend un groupe fonctionnel de fixation (A), un groupe d'espacement (B) et une fraction de détection de potentiel (PS) selon la formule I :
A-B-PS (I)
dans laquelle A est un groupe fonctionnel organique fixé à la nanostructure (104), B est un alkyle ou un aryle linéaire ou ramifié, substitué ou non substitué, et PS est un groupe fonctionnel organique capable de se lier à une molécule cible.
